# EUROPÄISCHE PATENTSCHRIFT

(11) **EP 0 513 299 B1**
(45) Veröffentlichungstag und Bekanntmachung des Hinweises auf die Patenterteilung: **24.05.1995**
(21) Anmeldenummer: 92900339.0
(22) Anmeldetag: 29.11.1991
(51) Int. Cl.: A61M 5/142

(54) **IMPLANTIERBARE INFUSIONSPUMPE**
IMPLANTABLE INFUSION PUMP
POMPE A PERFUSION IMPLANTABLE

(30) Priorität: 29.11.1990 DE 4038049
(43) Veröffentlichungstag der Anmeldung: 19.11.1992
(73) Patentinhaber: ANSCHÜTZ & CO. GmbH, 24106 Kiel (DE)
(72) Erfinder: OTTO, Karl-Heinz, D-2300 Kiel 14 (DE); PFISTER, Gerd, D-2300 Kiel 14 (DE)
(74) Vertreter: Tönnies, Jan G., Dipl.-Ing.
(86) Internationale Anmeldenummer: DE9100944
(87) Internationale Veröffentlichungsnummer: WO9209316

(56) Entgegenhaltungen:
- US-A- 4 715 852
- US-A- 4 838 887
- US-A- 4 857 055

## Beschreibung

Die Erfindung betrifft eine implantierbare Infusionspumpe mit einem elastischen Medikamentenbehälter, einem auf diesen wirkenden Treibgasraum mit einem Treibmittel aus einem FCKW und einer Kapillare zur Drosselung. Verschiedene Beispiele dieser Art von Infusionspumpe sind in US-A-4.857.055 oder in US-A-4.715.852 oder in US-A-4.838.887 gegeben.

Implantierbare Infusionspumpen werden zur Dauergabe von Medikamenten, z. B. Morphinen in einer gleichbleibenden Dosis über lange Zeiträume verwendet. Sie haben gegenüber gewohnlichen Injektionen den Vorteil, daß man eine Dosis nicht mehr soweit überdosieren muß, daß trotz Abbaus des Medikamentes bis zum nächsten Verabreichungszeitpunkt eine gewisse Mindestdosis nicht unterschritten wird, sondern man eine gleichmäßige und insgesamt wesentlich verringerte Zufuhr des Medikamentes verwirklichen kann.

Bisher ergeben sich jedoch bei Infusionspumpen der oben genannten Art Probleme, wenn sie einer veränderten Temperatur unterworfen werden. Falls die Person, die eine solche Infusionspumpe eingepflanzt bekommen hat, Fieber mit 42° Celsius Körpertemperatur bekommt, kann die Fördermenge aufgrund der zusätzlichen Ausdehnung des Treibmittels bis zu 46 % mehr betragen (Treibmittel : Frigen 11). Dies ist natürlich in hohem Maße unerwünscht.

Ähnliche Probleme können sich durch veränderten Luftdruck ergeben. Hier kann es, z. B. bei einem Aufenthalt in 3000 m Höhe, was in etwa dem Luftdruck entspricht, der in einem Flugzeug auftreten kann, sogar zu einer zusätzlichen Fördermenge von 55 % kommen.

Der Erfindung lag daher die Aufgabe zugrunde, eine implantierbare Infusionspumpe zu schaffen, bei der diese Effekte eine möglichst geringe Rolle spielen und die Betriebssicherheit der Pumpe nach Möglichkeit noch zu erhöhen.

Erfindungsgemäß wird dies dadurch erreicht, daß der Treibgasraum für einen Fülldruck von mehr als 2,5 bar über Atmosphärendruck eingerichtet ist, und das Treibmittel 1,2- Dichlortetrafluorethan ( C₂Cl₂F₄ ) ist.

Dieses Treibmittel besitzt eine für diesen Zweck sehr gut geeignete Dampfdruckkurve und ist ungiftig, also harmlos, falls es mit dem Körper in Berührung kommt.

Weiter wird vorgeschlagen, daß der Medikamentenbehälter aus einem Metallmembranfaltenbalg besteht. Dieser wiederum sollte vorzugsweise aus Titan gefertigt sein. Dieses Material ist leicht, chemisch resistent, genügend undurchlässig und mechanisch sehr widerstandsfähig.

In einer bevorzugten konstruktiven Ausgestaltung wird ein Außenbehälter den Treibgasraum und den Medikamentenbehälter aufnehmen. Dieser Außenbehälter sollte ebenfalls aus Titan gefertigt sein. Dies hat die gleichen Vorteile wie beim Medikamentenbehälter und zusätzlich noch den der großen Biokompatibilität von Titan.

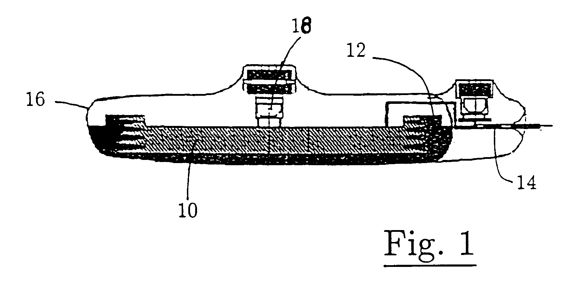

Im folgenden wird die Erfindung anhand einer Zeichnung näher erläutert. Dabei zeigt Fig. 1, die einzige Darstellung, eine implantierbare Infusionspumpe in Schnittdarstellung.

Die Infusionspumpe besteht aus einem Außenbehälter 16 in dem sich ein Medikamentenbehälter 10, der in Form eines Metallmembranfaltenbalges ausgebildet ist, ein Treibgasraum 12, eine Kapillare 14 zur Drosselung des Medikamentflusses und ein Septum 18, das der Zuführung des Medikamentes durch einen Arzt mit Hilfe einer Spritze mit einer speziellen Nadel dient, befinden.

Die gesamte dargestellte Pumpe wird einem Patienten bevorzugt in der Bauchregion implantiert. Von der Infusionspumpe geht dann ein Katheter an den Ort, wo die Medikamentengabe geschehen soll, z. B. an die Wirbelsäule.

Je nach gewählter Fördermenge und Größe der Pumpe wird sie nur in langen Zeiträumen, typisch ist ein Monat, zu befüllen sein. Dazu werden spezielle Nadeln verwandt, die spanlos durch das Septum 18, eine Art Korken, hindurchtreten können.

Der hohe Druck, unter dem das Treibmittel steht, bewirkt, daß sich Temperaturänderungen bei dem gewählten Treibmittel nur noch halb so stark äußern wie zuvor, und Druckschwankungen sogar nur noch ungefähr eine Viertel der vorher erreichbaren Toleranzen in der Fördermenge ausmachen.

Ein sehr erwünschter weiterer Effekt ist, daß sich durch den hohen Druck außer der genaueren Dosierung eine höhere Betriebssicherheit ergibt, weil die Kapillare nun mit einem höheren Druck beaufschlagt ist, und Partikel in dem Medikament noch weniger den Durchfluß stören können.

Zwischen dem Medikamentenbehälter und der Kapillare ist noch ein Membranfilter vorgesehen, der von einem Stützsieb getragen wird.

Aus Festigkeitsgründen, aber auch weil das Material chemisch sehr resistent und biokompatibel ist, und zusätzlich noch leicht ist, wird für alle Teile, bei denen es auf Stabilität ankommt, Titan verwandt.

Der Außenbehälter 16 bildet direkt den Treibgasraum 12, also den Druckbehälter für das Treibmittel. Beim Befüllen des Medikamentenbehälters 10 wird sich dieser in dem Treibgasraum 12 ausdehnen und das Treibmittel verflüssigen. Umgekehrt wird sich das Treibmittel sehr langsam wieder in die Gasphase umwandeln, wenn ihm durch das ausgetriebene Medikament mehr Platz zur Verfügung steht.

## Patentansprüche

1. Implantierbare Infusionspumpe mit einem elastischen Medikamentenbehälter (10), einem auf diesen wirkenden Treibgasraum (12) mit einem Treibmittel aus einem FCKW und einer Kapillare (14) zur Drosselung,
dadurch gekennzeichnet, daß
der Treibgasraum für einen Fülldruck von mehr als 2,5 bar über Atmosphärendruck eingerichtet ist, und das Treibmittel 1,2- Dichlortetrafluorethan ( C₂Cl₂F₄ ) ist.

2. Implantierbare Infusionspumpe nach Anspruch 1, dadurch gekennzeichnet, daß der Medikamentenbehälter (10) aus einem Metallmembranfaltenbalg besteht.

3. Implantierbare Infusionspumpe nach Anspruch 2, dadurch gekennzeichnet, daß der Medikamentenbehälter (10) aus Titan besteht.

4. Implantierbare Infusionspumpe nach einem der vorangehenden Ansprüche, gekennzeichnet durch einen den Treibgasraum (12) und den Medikamentenbehälter (10) aufnehmenden Außenbehälter (16).

5. Implantierbare Infusionspumpe nach einem der vorangehenden Ansprüche, dadurch gekennzeichnet, daß der Außenbehälter (16) aus Titan gefertigt ist.

## Claims

1. Implantable infusion pump with an elastic medicament container (10), a propellant gas chamber (12) acting thereon with a propellant formed from a fluorocarbon and a restricting capillary (14), characterized in that the propellant gas chamber is designed for a filling pressure of more than 2.5 bar above atmospheric pressure and the propellant is 1,2-dichlorotetrafluoroethane (C₂Cl₂F₄).

2. Implantable infusion pump according to claim 1, characterized in that the medicament container (10) comprises a metal membrane bellows.

3. Implantable infusion pump according to claim 2, characterized in that the medicament container (10) is made from titanium.

4. Implantable infusion pump according to any one of the preceding claims, characterized by the outer container (16) receiving the propellant gas chamber (12) and the medicament container (10).

5. Implantable infusion pump according to any one of the preceding claims, characterized in that the outer container (16) is made from titanium.

## Revendications

1. Pompe d'infusion implantable munie d'un réservoir à médicaments élastique (10), d'un espace contenant un gaz propulseur (12), qui agit sur le réservoir à l'aide d'un agent de propulsion d'hydrocarbures chlorofluorés (HCF) et d'un capillaire pour l'étranglement,
caractérisée par le fait que:
l'espace contenant le gaz propulseur est aménagé de telle façon à pouvoir résister à une pression de remplissage supérieure à 2,5 bar au-dessus de la pression atmosphérique et l'agent de propulsion est le 1,2- di-chloro-tétra-fluoro-éthane (C₂Cl₂F₄).

2. Une pompe d'infusion, selon la revendication figurant sous le chiffre 1, qui est caractérisée par le fait que le réservoir contenant le médicament (10) est composé d'un soufflet d'intercirculation à membrane métallique.

3. Une pompe d'infusion, selon la revendication figurant sous le chiffre 2, qui est caractérisée par le fait que le réservoir contenant le médicament (10) est en titane.

4. Une pompe d'infusion, selon l'une des revendications précédentes, caractérisée par un réservoir extérieur (16) qui héberge l'espace contenant le gaz propulseur (12) ainsi que le réservoir contenant le médicament (10).

5. Une pompe d'infusion, selon l'une des revendications précédentes, qui est caractérisée par le fait que le réservoir extérieur (16) est en titane.
